# EUROPÄISCHE PATENTSCHRIFT

(11) **EP 2 010 500 B1**
(45) Veröffentlichungstag und Bekanntmachung des Hinweises auf die Patenterteilung: **08.06.2011**
(21) Anmeldenummer: 07727781.2
(22) Anmeldetag: 04.04.2007
(51) Int. Cl.: H01L 23/40, H05K 7/20

(54) **ANORDNUNG ZUR KONTAKTIERUNG VON LEISTUNGSHALBLEITERN AN EINER KÜHLFLÄCHE**
ARRANGEMENT FOR CONTACTING POWER SEMICONDUCTORS TO A COOLING SURFACE
DISPOSITIF DE CONTACT DE SEMI-CONDUCTEURS DE PUISSANCE SUR UNE SURFACE RÉFRIGÉRANTE

(30) Priorität: 20.04.2006 DE 102006018716
(43) Veröffentlichungstag der Anmeldung: 07.01.2009
(73) Patentinhaber: ebm-papst Mulfingen GmbH & Co. KG, 74673 Mulfingen (DE)
(72) Erfinder: MASCHKE, Matthias, 74523 Schwäbisch Hall (DE); SCHNEEWEISS, Hartmut, 97980 Bad Mergentheim (DE); ULMER, Anja, 74572 Blaufelden (DE)
(74) Vertreter: Patentanwälte Dr. Solf & Zapf
(86) Internationale Anmeldenummer: PCT/EP2007/053310
(87) Internationale Veröffentlichungsnummer: WO 2007/122084

(56) Entgegenhaltungen:
- EP-A1- 0 788 155
- EP-A2- 1 130 744
- DE-A1- 10 317 182
- FR-A- 2 780 456
- US-A- 5 274 193
- US-A- 5 466 970
- US-A- 5 909 358

## Beschreibung

Die vorliegende Erfindung betrifft eine Anordnung zur Kontaktierung von mindestens einem in einem Gehäuse mit Anschlussdrähten montiertem elektronischen Bauelement, insbesondere einem Leistungshalbleiter, an einer Kühlfläche und einem im Gehäuse befestigbaren Federelement mit mindestens einem Federarm, der in einer Andruckposition des Federelements das Bauelement gegen die Kühlfläche drückt.

Um die Kühlung von Leistungshalbleitern zu gewährleisten, werden diese in wärmeleitenden Kontakt zu einem Kühlkörper gesetzt. Dies wird in kostengünstigen Ausführungen meist mit Federn realisiert, die das Halbleiterbauelement an den Kühlkörper pressen.

Eine solche Anordnung ist in der deutschen Gebrauchsmusterschrift G 92 13 671.0 beschrieben. Durch die Vormontage eines Haltefederelements in einem Gehäuseteil werden bei anschließendem Zusammensetzen der Gehäuseteile die zu kühlenden Bauelemente durch Federzungen der Haltefeder gegen eine Innenwand des Kühlkörpers gedrückt.

In ähnlicher Weise zeigen die US-Patentschrift 5,274,193, die deutsche Offenlegungsschrift 36 12 862 A1, die deutsche Patentschrift 195 43 260 C2 und die deutsche Gebrauchsmusterschrift DE 200 14 739 U1 Federelement, die zwischen Leistungshalbleitern und Gehäuse verklemmt werden. Unterschiedlich ist jeweils die Abstützung bzw. die Befestigung der Federelemente an dem Gehäuse. So kann beispielsweise das Federelement selbst durch Ausnehmungen und Stege des Gehäuses gehalten werden oder mittels zusätzlicher Befestigungselemente wie Schrauben oder Klemmschienen gehalten werden.

Dem Stand der Technik gehören weiterhin Haltevorrichtungen an, die im Wesentlichen einen U- oder L-förmigen Federbügel aufweisen, der über eine Kühlkörperwandung und gleichzeitig über das Bauelement gesteckt ("geclipt") wird und auf diese Weise die Andruckkraft zwischen den Anlageflächen des Kühlkörpers und des Bauelements sicherstellt.

Bei allen Vorrichtungen ohne zusätzliche Befestigungselemente, aus dem deutschen Gebrauchsmuster G 92 13 671.0 bekannt, tritt das Problem auf, dass das Bauteil und die Lötstelle durch Schubspannungen belastet werden. Dies kann zu einer Schädigung des Bauteils oder der Lötverbindung führen. Die Schubspannungen entstehen durch Kräfte, die beim Zusammenbau der Vorrichtung in einer Ebene parallel zur Kohlanlagefläche der Bauelemente bzw. des Kühlkörpers auf das Bauelement einwirken.

In dem im US-Patent 5,274,193 offenbarten Gegenstand wirkt zwar keine Schubkraft auf die Bauelemente, die Fixierung der Haltefeder und das Erzeugen der Haltekraft des Federelements muss aber durch eine zusätzliche Klemmschiene bewerkstelligt werden.

Aus der US 5,909,358 ist eine gattungsgemäße Anordnung bekannt. Bei dieser bekannten Anordnung muss das Federelement beim Übergang in die Andruckposition eine Art Schwenkbewegung durchführen und zudem erfolgt die Montage des Federelements senkrecht zur Kühlfläche, so dass ein relativ großer Platzbedarf vor der Kühlfläche für das Einführen des Federelements erforderlich ist

Eine entsprechende Montage erfolgt auch bei dem Federelement, wie es aus der DE 103 17182 A1 bekannt ist.

Auch das aus der FR 2780456 bekannte Federelement wird senkrecht zur Kühlfläche montiert, so dass auch hier der vor der Kühlfläche erforderliche freie Montageraum vorhanden sein muss, um eine ordnungsgemäße Montage vornehmen zu können.

Der vorliegenden Erfindung liegt die Aufgabe zu Grunde, eine Anordnung der eingangs beschriebenen Art zu schaffen, die bei einfacher Montage keine Schubkräfte in Richtung oder Gegenrichtung der Anschlussdrähte für die elektrischen Bauelemente ausübt.

Eine Anordnung gemäß dem Oberbegriff des Patentanspruchs 1 ist aus US 5,909,358 bekannt.

Erfindungsgemäß wird die oben erwähnte Aufgabe dadurch erreicht, dass die Gehäusewandung in den an die als Kühlfläche wirkende seitlichen Wand angrenzenden Seitenwänden senkrecht nach unten auf den Boden zu verlaufende eingelassene Nutenpaare aufweist, die jeweils aus einer Einfügenut und einer Rastnut bestehen, wobei die Einfügenuten deart sind, dass das Federelement in axialer Richtung der Einfügenuten gegenüber dem Leistungshalbleiter berührungsfrei parallel zur Kühlfläche in einer Einfügeposition mittels seitlich an einem Trägerelement des Federelements abgewinkelter elastischer Ansätze einschiebbar ist, und die Rastnuten mit den Einfügenuten über derart angeformte Übergangsbereiche verbunden sind, dass das in der Einfügeposition befindliche Federelement mittels der Ansätze durch eine senkrecht zur Kühlfläche wirkenden Kraft in die Rastnuten in die Andruckposition rastend verschiebbar ist und die Rastnuten derart ausgebildet sind, dass das Federelement in der Andruckposition halten.

Die Rastnuten ermöglichen ein zweckmäßiges automatisches Einrasten des Federelements in vordefinierter Position. Eine aufwändige Ausrichtung des Federelements entfällt.

Das Federelement ist von der ersten Montageposition durch eine Kraftwirkung senkrecht zur Kühlfläche in die Andruckposition verschiebbar, so dass bei diesem zweiten Montageschritt keine Schubkraft in Längsrichtung der Anschlussdrähte wirkt, statt dessen wird das Federelement aus der ersten Montageposition heraus durch rein senkrecht auf die Kühlfläche wirkende Kräfte in der Andruckposition fixiert.

In weiterer vorteilhafter Ausgestaltung besteht das Federelement aus parallel zueinander verlaufenden Federarmen, die senkrecht an einem Trägerelement befestigt sind, das die beiden seitlichen Ansätze des Federelements miteinander verbindet Diese Ausführung erlaubt es auf Grund der Steifigkeit hohe Anpresskräfte aufzubringen, wobei vorteilhafterweise nur ein Federelement für alle gleichförmig angeordneten Halbleiterbauelemente erforderlich ist.

Weitere zweckmäßige Ausgestaltungsmerkmale werden an Hand eines bevorzugten, in den Zeichnungen veranschaulichten Ausführungsbeispiels genau erläutert. Dabei zeigen:
- Fig. 1: eine Vorderansicht eines erfindungsgemäßen Federelements,
- Fig. 2: eine rückwärtige Ansicht des erfindungsgemäßen Federelements,
- Fig. 3: das Federelement vor dem Einsatz in ein Elektronikgehäuse,
- Fig. 4: das Federelement in einer ersten Montageposition,
- Fig. 5: das Federelement in eingerasteter Andruckposition,
- Fig. 6: Detailansicht des Federelements in der ersten Montageposition,
- Fig. 7: Detailansicht des Federelements in eingerasteter Andruckposition.

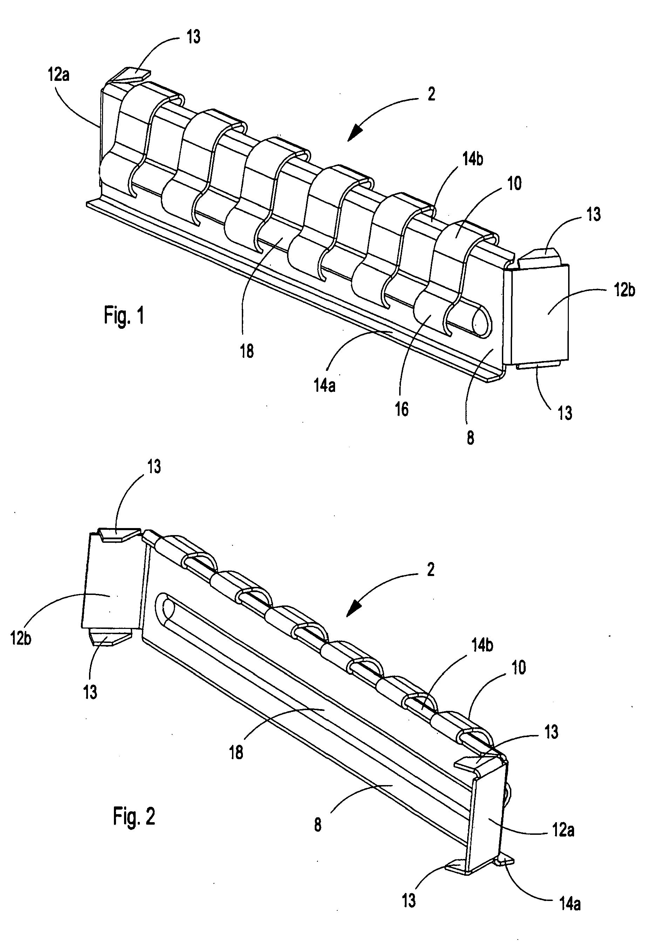

Die Fig. 1 und 2 zeigen ein erfindungsgemäßes Federelement 2 wie es zum Einbau in ein Elektronikgehäuse 4 eines Elektromotors vorgesehen ist. Das Federelement 2 besteht aus einem Trägerelement 8, an dessen oberer Kante vorliegend z. B. sechs Federarme 10 angeformt sind und das seitlich zwei abgewinkelte Ansätze 12a, 12b aufweist. Die Ansätze 12a, 12b sind an ihrer oberen und unteren Kante mit rechtwinklig nach innen umgefalzten Laschen 13 ausgebildet, um ein Einknicken der Ansätze 12a, 12b zu verhindern. An der Ober- und Unterkante besitzt das Trägerelement ebenfalls etwa rechtwinklig umgefalzte schmale Abschnitte 14a, 14b, wobei der untere Abschnitt 14a zu der Seite weist, an der sich die Federarme 10 befinden und der obere Abschnitt 14b in die entgegengesetzte Richtung weist. Der obere Abschnitt 14b setzt sich in äquidistanten Abständen fort (Fig. 2) und bildet über die Oberkante des Trägerelements 8 nach unten in Richtung auf den Abschnitt 14 a gebogen die parallel zueinander verlaufenden Federarme 10. An ihrem unteren Ende besitzen die Federarme 10 einen nach außen konvex gekrümmten Kontaktbereich 16, der in Andruckposition des Federelements 2 gegen die Bauelemente 24 drückt. Unterhalb der Mitte des Trägerelements 8, gegenüberliegend der Kontaktbereiche 16, verläuft parallel zur Ober- und Unterkante des Trägerelements 8 eine zur Vorderseite hin gewölbte, halbkreisförmige Versteifungssicke 18, die ein Durchbiegen des Federelements 2 verhindert.

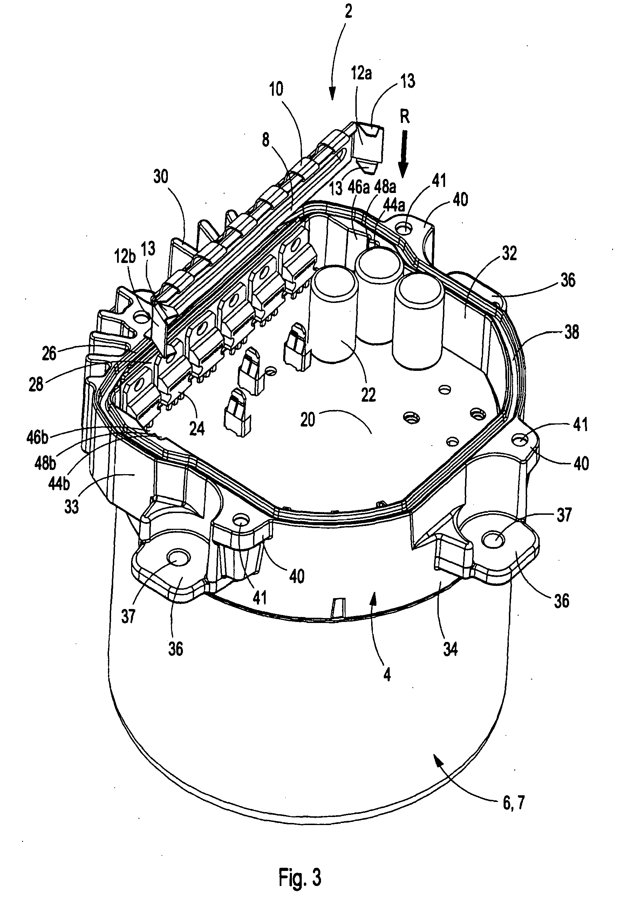

Die Fig. 3 bis 7 zeigen eine mögliche Verwendungsvariante des erfindungsgemäßen Federelementes 2 in einem Elektronikgehäuse 4 eines Elektromotors 6. Auf ein Rotorgehäuse 7 des Elektromotors 6 ist das Elektronikgehäuse 4 aufgesetzt, das eine Leiterplatte 20 mit elektronischen Bauelementen 22 sowie weitere elektrische Verbindungselemente aufnimmt (Fig. 3). Das Elektronikgehäuse 4 besteht im Wesentlichen aus einer Umfangswandung, die von einer ebenen seitlichen Wand 26, die als Kühlfläche wirkt, daran angrenzenden Seitenwänden 32, 33 und einer der ebenen seitlichen Wand 26 gegenüberliegenden Wand 34 gebildet wird. An den Seitenwänden 32, 33 sowie an der gegenüberliegenden Wand 34 sind Halterungen 36 mit Bohrungen 37 zur Befestigung des Gehäuses angeformt. Das Elektronikgehäuse 4 kann mit einem nicht dargestellten Gehäusedeckel verschlossen werden, der in eine umlaufende Nut 38 an der Stirnseite der Seitenwände 26, 32, 33 und 34 eingreift. Der Gehäusedeckel ist an Haltevorsprüngen 40, die Bohrungen 41 aufweisen, befestigt. Auf dem Boden des Elektronikgehäuses 4 sitzt eine die elektronischen Bauelemente 22, 24 tragende Leiterplatte 20. Dabei sind Leistungshalbleiter 24 so auf der Leiterplatte 20 eingelötet, dass sie im Innern des Elektronikgehäuses 4 an einer ebenen seitlichen Wand 26 des Elektronikgehäuses 4 angeordnet werden können. Zwischen dem Leistungshalbleiter 24 und der ebenen Seitenwand 26 ist eine wärmeleitende elektrische Isolierschicht 28 eingefügt, um einen Wärmetransport zu der mit Kühlrippen 30 versehenen Seitenwand 26 zu ermöglichen und zu verbessern.

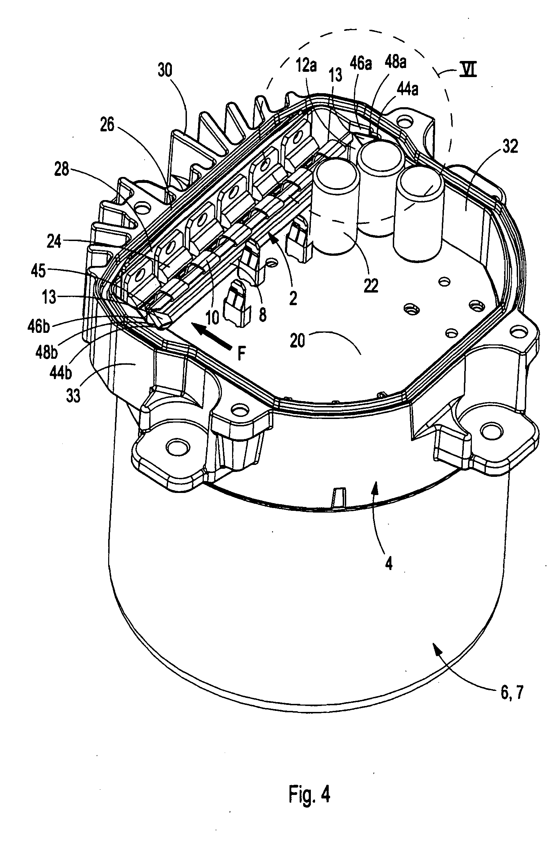

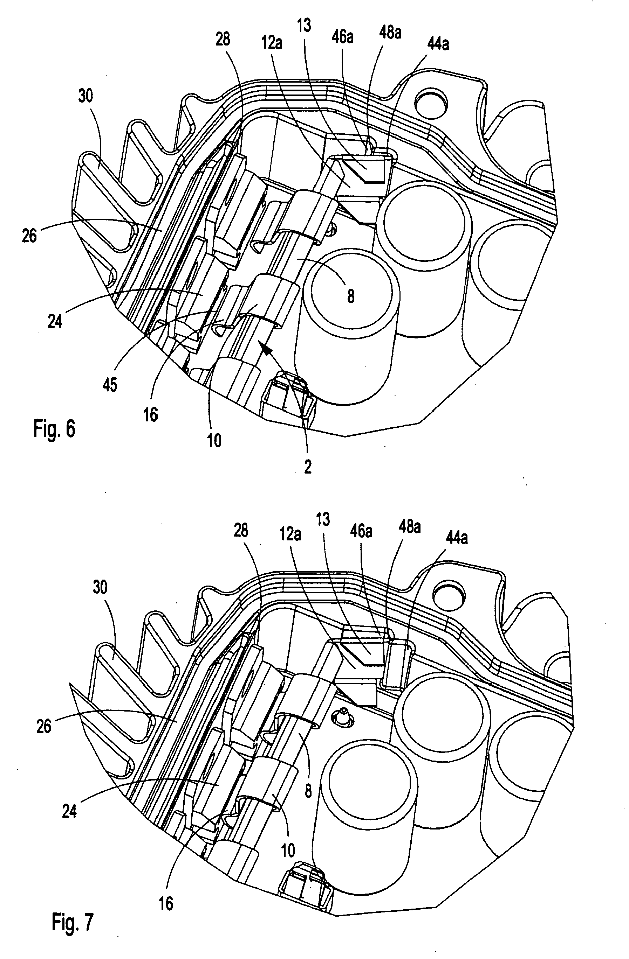

In die an die ebene Seitenwand 26 angrenzenden Seitenwände 32 und 33 sind senkrecht nach unten verlaufende Nutpaare eingelassen, die die Funktion als Einfügenuten 44a, 44b und als Rastnuten 46a, 46b erfüllen. Die zwei Einfügenuten 44a, 44b befinden sich beiseitig der ebenen Seitenwand 26 in jeweils gleichem Abstand von dieser einander gegenüber in den Seitenwänden 32, 33. In diese Einfügenuten 44a, 44b wird das Federelement 2 axial entsprechend der Pfeilrichtung R in Fig. 3 eingeschoben und nimmt eine erste Montageposition (Einfügeposition) ein. Die an der Oberseite angebrachten Laschen 13 können dabei als Angriffsflächen für die per Hand aufgebrachten Einschubkräfte dienen. Neben dieser Funktion als Montagehilfe besitzt das obere Laschenpaar 13 noch eine zusätzliche Aufgabe: Wenn das *Federelement 2 in das Elektronikgehäuse 4 eingesetzt, aber noch nicht verrastet ist, lässt sich ein Gehäusedeckel nicht montieren, da am Gehäusedeckel angespritzte Zapfen dann auf den oberen Laschen 13 aufliegen und ein Schließen des Deckels verhindern würden. Ist die ordnungsgemäße Verrastung des Federelementes 2 durchgeführt, kann der Zapfen des Deckels in den frei gewordenen Raum eingreifen und der Deckel lässt sich aufsetzen. Man hat hierdurch eine Kontrolle, ob eine ordnungsgemäße Montage des Federelementes 2 durchgeführt wurde. Die Einfügeposition ist in Fig. 4 dargestellt. Eine Detailansicht der Lage des Federelements in dieser Einfügeposition zeigt Fig. 6. Man erkennt, dass der Ansatz 12a durch Anlage seines Endbereichs in der Einfügenut 44a fixiert ist. Bei dem Einschubvorgang kommen die Federarme 10 noch nicht mit den Leistungshalbleitern 24 in Berührung, üben somit keinerlei Schubkräfte auf das Bauteil 24 und dessen Lötstelle aus. Es verbleibt ein Luftspalt 45 zwischen den Federarmen 10 und den Leistungshalbleitern 24.

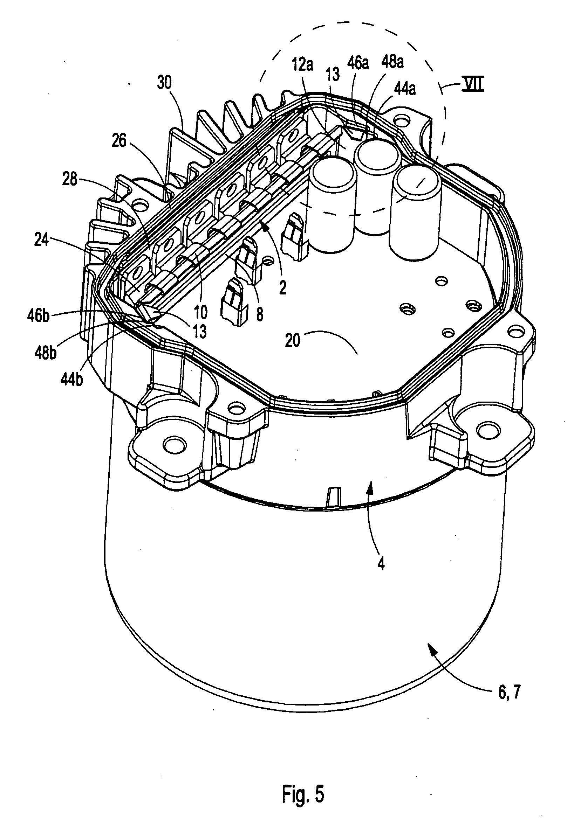

Das Rastnutpaar 46a, 46b schließt sich unmittelbar an die Einfügenuten 44a, 44b an und ist in gleicher Weise, aber mit geringerem Abstand zur ebenen Seitenwand 26 eingelassen. Der Übergangsbereich 48a, 48b zwischen Einfügenut 44a (44b) und Rastnut 46a (46b) ist derart ausgeformt, dass dessen Widerstand durch eine Krafteinwirkung F auf das Federelement 2 senkrecht zur Kühlfläche überwunden werden kann (Fig. 4). Dabei werden die elastischen seitlichen Ansätze 12a, 12b kurzzeitig bei der Vorschubbewegung in Richtung des Leistungshalbleiters 24 durch die Übergangsbereiche 48a, 48b zusammengedrückt, bevor sie in den Rastnuten 46a, 46b in der Andruckposition anliegen (Fig. 5).

Eine Detailansicht der Lage des Federelements 2 an der Seitenwand 32 in Andruckposition zeigt Fig. 7. Der Endbereich des Ansatzes 12a fixiert mit seiner senkrecht verlaufenden Kante, die jetzt in die Rastnut 46a eingebettet ist, das Federelement 2. In dieser Andruckposition drücken nun die Federarme 10 auf die Leistungshalbleiter 24, die dadurch gegen die wärmeleitende Isolierschicht 28 und damit gegen die als Kühlfläche 27 ausgebildete Seitenwand 26 gepresst werden. Dadurch wird ohne Verwendung weiterer Verbindungselemente, wie Schrauben oder Klemmen, eine sichere Wärmeableitung gewährleistet. Das Bauteil 24 und die Lötstelle werden nicht durch Schubkräfte belastet.

Die Erfindung ist nicht auf die dargestellten und beschriebenen Ausführungsbeispiele beschränkt, sondern umfaßt auch alle im Sinne der Erfindung gleichwirkenden Ausführungen.

## Patentansprüche

1. Anordnung zur Kontakberung von mindestens einem elektronischen Bauelement (24), insbesondere einem Leistungshalbleiter (24), an einer Kühlfläche (26), bestehend aus einem Gehäuse (4), an dessen Kühlfläche (26) das elektronische Bauelement (24) mit Anschlussdrähten montiert ist und aus einem im Gehäuse (4) befestigbaren Federelement (2) mit mindestens einem Federarm (10), der in einer Andruckposition des Federelements (2) das Bauelement (24) gegen die Kühlfläche (26) drückt, und das Federelement (2) ohne Berührung des Bauelements (24) in das Gehäuse (4) einführbar ist und das Gehäuse (4) derart mit Haltemitteln (46a, 46b) für das Federelement (2) versehen ist, dass bei der Montage des Federelements (2) keine Schubkräfte in Längsrichtung der Anschlussdrähte auftreten,
**dadurch gekennzeichnet, dass** die Gehäusewandung in den an die als Kühlfläche wirkenden seitlichen Wand (26) angrenzenden Seitenwänden (32, 33) senkrecht nach unten auf den Boden zu verlaufende, eingelassene Nutenpaare aufweist, die jeweils aus einer Einfügenut (44a, 44b) und einer Rastnut (46a, 46b) bestehen, wobei die Einfügenuten (44a, 44b) derart sind, dass das Federelement (2) in axialer Richtung (R) der Einfügenuten gegenüber dem Leistungshalbleiter (24) berührungsfrei parallel zur Kühlfläche (26) in einer Einfügeposition mittels seitlich an einem Trägerelement des Federelements (2) abgewinkelter elastischer Ansätze (12a, 12b) einschiebbar ist, und die Rastnuten (46a, 46b) mit den Einfügenuten (44a, 44b) über derart angeformte Übergangsbereiche (48a, 48b) verbunden sind, dass das in der Einfügeposition befindliche Federelement (2) mittels der Ansätze (12a, 12b) durch eine senkrecht zur Kühlfläche (26) wirkenden Kraft (F) in die Rastnuten (46a, 46b) in die Andruckposition rastend verschiebbar ist, und die Rastnuten (46a, 46b) derart ausgebildet sind, dass sie das Federelement (2) in der Andruckposition halten.

2. Anordnung nach Anspruch 1,
**dadurch gekennzeichnet, dass** mehrereFederarme(10)parallel zueinander verlaufend an der im Montagezustand der Leiterplatte abgewandten Kante des Trägerelements (8) befestigt sind.

3. Anordnung nach einem der Ansprüche 1 bis 2,
**dadurch gekennzeichnet, dass** das Trägerelement (8) eine zwischen beiden seitlichen Ansätzen (12a, 12b) verlaufende Versteifungssicke (18) aufweist.

4. Anordnung nach einem der Ansprüche 1 bis 3.
**dadurch gekennzeichnet, dass** das Trägerelement (8) an seinen Längsseiten rechtwinklig umgefalzte Abschnitte (14a, 14b) aufweist.

5. Anordnung nach einem der Ansprüche 1 bis 4,
**dadurch gekennzeichnet, dass** die seitlichen Ansätze (12a, 12b) des Federelements (2) rechtwinklig angeformte Laschen (13) aufweisen.

6. Anordnung nach einem der Ansprüche 1 bis 5,
**dadurch gekennzeichnet, dass** mehrere in einer Reihe mit gleicher Ausrichtung angeordnete Bauelemente (24) mit einem Federelement (2) angedrückt werden.

7. Anordnung nach einem der Ansprüche 1 bis 6,
**dadurch gekennzeichnet, dass** die als Kühlfläche (26) ausgebildete seitliche Wand (26) eine in das Gehäuseinnere weisende planparallele Anlagefläche aufweist und an ihrer Außenseite mit Kühlrippen (30) versehen ist.

8. Anordnung nach einem der Ansprüche 1 bis 6,
**dadurch gekennzeichnet, dass** eine wärmeleitende Isolierschicht (28) zwischen der Kühlfläche (26) und dem Bauelement (24) angebracht ist.

## Claims

1. An arrangement for the contacting of at least one electronic component (24), in particular a power semiconductor (24), against a cooling surface (26), consisting of a housing (4), on whose cooling surface (26) there is mounted the electronic component (24) by means of connecting wires, and a spring member (2), securable in the housing (4), with at least one spring arm (10) which, in a pressure position of the spring element (2), presses the component (24) against the cooling surface (26), and the spring element (2) is insertable into the housing (4) without touching the component (24) and the housing (4) is provided with holding means (46a, 46b) for the spring element (2) in such a manner that upon mounting of the spring element (2) there is no occurrence of transverse forces in the longitudinal direction of the connecting wires, **characterised in that** the housing wall has let-in groove pairs, running perpendicularly downwards toward the base, in the side walls (32, 33) adjoining the lateral wall (26) acting as a cooling surface, which groove pairs each consist of an insertion groove (44a, 44b) and a locking groove (46a, 46b), wherein the insertion grooves (44a, 44b) are such that the spring element (2) can, by means of resilient extensions (12a, 12b) laterally bent at an angle on a bearer element of the spring element, in an insertion position be inserted in the axial direction (R) of the insertion grooves, in a contact-free manner with respect to the power semiconductor (24), parallel to the cooling surface (26), and the locking grooves (46a, 46b) are connected to the insertion grooves (44a, 44b) via transition regions (48a, 48b) formed in such a manner that the spring element (2) located in the insertion position is, through a force (F) acting perpendicular to the cooling surface (26), displaceable in a locking manner into the locking grooves (46a, 46b) into the pressure position by means of the extensions (12a, 12b), and the locking grooves (46a, 46b) are such that they hold the spring element (2) in the pressure position.

2. An arrangement according to claim 1, **characterised in that** a plurality of springs arms (10) are secured, so as to run parallel to one another, to the edge, remote from the printed circuit board in the mounted state, of the bearer element (8).

3. An arrangement according to one of claims 1 to 2, **characterised in that** the bearer element (8) has a stiffening depression (18) running between both lateral extensions (12a, 12b).

4. An arrangement according to any one of claims 1 to 3, **characterised in that** the bearer element (8) has, on its long sides, portions (14a, 14b) which are folded over at a right angle.

5. An arrangement according to any one of claims 1 to 4, **characterised in that** the lateral extensions (12a, 12b) of the spring element (2) have flaps (13) formed thereon at a right angle.

6. An arrangement according to any one of claims 1 to 5, **characterised in that** one spring element (2) presses against a plurality of components (24) arranged in a row and with the same orientation.

7. An arrangement according to any one of claims 1 to 6, **characterised in that** the lateral wall (26) in the form of a cooling surface (26) has a plane-parallel contact surface directed into the housing interior and is provided with cooling ribs (30)at its exterior.

8. An arrangement according to any one of claims 1 to 6, **characterised in that** a heat-conducting insulating layer (28) is fitted between the cooling surface (26) and the component (24).

## Revendications

1. Dispositif pour la mise en contact d'au moins un composant électronique (24), en particulier un semi-conducteur de puissance (24), sur une surface de refroidissement (26), formé par un boîtier (4), sur la surface de refroidissement (26) duquel le composant électronique (24) est monté avec des fils de raccordement, et par un élément de ressort (2), apte à être fixé dans le boîtier (4) et comportant au moins un bras de ressort (10) qui, dans une position de pression de l'élément de ressort (2), pousse le composant (24) contre la surface de refroidissement (26), et l'élément de ressort (2) peut être introduit dans le boîtier (4) sans entrer en contact avec le composant (24), et le boîtier (4) est muni de moyens de fixation (46a, 46b) pour l'élément de ressort (2), de telle sorte qu'aucune force de poussée ne se produit dans le sens longitudinal des fils de raccordement au moment du montage de l'élément de ressort (2),
**caractérisé en ce que** la paroi du boîtier, dans les parois latérales (32, 33) adjacentes à la paroi latérale (26) faisant fonction de surface de refroidissement, comporte des paires de rainures noyées, qui s'étendent perpendiculairement vers le bas vers le fond et qui sont formées par une rainure d'insertion (44a, 44b) et une rainure de blocage (46a, 46b), les rainures d'insertion (44a, 44b) étant telles que l'élément de ressort (2) peut être inséré dans la direction axiale (R) des rainures d'insertion sans entrer en contact avec le semi-conducteur de puissance (24) parallèlement à la surface de refroidissement (26) dans une position d'insertion, au moyen de saillies (12a, 12b) élastiques, pliées latéralement sur un élément de support de l'élément de ressort, et les rainures de blocage (46a, 46b) sont reliées aux rainures d'insertion (44a, 44b) par des zones de transition (48a, 48b) formées de telle sorte que l'élément de ressort (2) situé dans la position d'insertion peut être déplacé dans la position de pression bloquée, au moyen des saillies (12a, 12b) sous l'effet d'une force (F) agissant perpendiculairement à la surface de refroidissement (26), et les rainures de blocage (46a, 46b) sont réalisées de telle sorte qu'elles maintiennent l'élément de ressort (2) dans la position de pression.

2. Dispositif selon la revendication 1, **caractérisé en ce que** plusieurs bras de ressort (10) sont fixés parallèlement entre eux sur le bord de l'élément de support (8), détourné de la plaquette de circuits imprimés dans la position de montage de celle-ci.

3. Dispositif selon l'une quelconque des revendications 1 à 2, **caractérisé en ce que** l'élément de support (8) comporte une nervure de raidissement (18) qui s'étend entre les deux saillies latérales (12a, 12b).

4. Dispositif selon l'une quelconque des revendications 1 à 3, **caractérisé en ce que** l'élément de support (8) comporte sur ses côtés longitudinaux des segments (14a, 14b) repliés perpendiculairement.

5. Dispositif selon l'une quelconque des revendications 1 à 4, **caractérisé en ce que** les saillies latérales (12a, 12b) de l'élément de ressort (2) comportent des pattes (13) formées perpendiculairement sur celles-ci.

6. Dispositif selon l'une quelconque des revendications 1 à 5, **caractérisé en ce que** plusieurs composants (24), agencés avec la même orientation en une rangée, sont pressés par un élément de ressort (2).

7. Dispositif selon l'une quelconque des revendications 1 à 6, **caractérisé en ce que** la paroi latérale (26), réalisée sous forme de surface de refroidissement (26), comporte une surface d'appui plane et parallèle, dirigée vers l'intérieur du boîtier, et comporte des ailettes de refroidissement (30) sur sa face extérieure.

8. Dispositif selon l'une quelconque des revendications 1 à 6, **caractérisé en ce qu'**une couche isolante (28) électroconductrice est déposée entre la surface de refroidissement (26) et le composant (24).
